**Europäisches Patentamt**

**European Patent Office**

**Office européen des brevets**

(19)

(11) Veröffentlichungsnummer: **0 235 133**
**B1**

(12) # EUROPÄISCHE PATENTSCHRIFT

(45) Veröffentlichungstag der Patentschrift:
**18.10.89**

(51) Int. Cl.⁴: **A 61 F 5/46, A 61 M 31/00**

(21) Anmeldenummer: **85905592.3**

(22) Anmeldetag: **13.11.85**

(86) Internationale Anmeldenummer:
**PCT/AT 85/00047**

(87) Internationale Veröffentlichungsnummer:
**WO 86/02826 (22.05.86 Gazette 86/11)**

(54) **INTRAVAGINAL POSITIONIERBARE, INSBESONDERE EMPFÄNGNISVERHÜTENDE VORRICHTUNG.**

(30) Priorität: **15.11.84 AT 3622/84**
**02.07.85 AT 1956/85**

(43) Veröffentlichungstag der Anmeldung:
**09.09.87 Patentblatt 87/37**

(45) Bekanntmachung des Hinweises auf die Patenterteilung:
**18.10.89 Patentblatt 89/42**

(84) Benannte Vertragsstaaten:
**BE FR**

(56) Entgegenhaltungen:
**CH-A- 429 020**
**DE-C- 844 045**
**FR-A- 335 672**
**FR-A- 534 946**
**FR-A- 2 484 247**
**US-A- 2 391 343**
**US-A- 3 128 762**
**US-A- 3 228 398**
**US-A- 3 786 807**
**US-A- 3 831 605**

(73) Patentinhaber: **PICKHARD, Ewald,**
**Redtenbachergasse 15, A-1160 Wien (AT)**
Patentinhaber: **Knogler, Wolfgang, Dr., Görgengasse 27,**
**A-1190 Wien (AT)**

(72) Erfinder: **PICKHARD, Ewald, Redtenbachergasse 15,**
**A-1160 Wien (AT)**
Erfinder: **Knogler, Wolfgang, Dr., Görgengasse 27,**
**A-1190 Wien (AT)**

(74) Vertreter: **Wolke, Heidemarie, Dr., Stadtplatz 7,**
**A-4400 Steyr (AT)**

## Beschreibung

Die Erfindung betrifft eine intravaginal positionierbare, insbesondere empfängnisverhütende Vorrichtung nach dem Oberbegriff des Patentanspruches 1 eine Manipulationsvorrichtung zur Durchführung des Verfahrens.

Derartige antikonzeptive Schwämme zählen zu den ältesten Methoden der Emfpängnisverhütung und es sind aus dem Altertum intravaginale Schwämmchen, welche mit Essig oder Öl getränkt wurden, bekannt geworden. Bei neuerdings vorgeschlagenen Einrichtungen der eingangs genannten Art wurde Essig oder Öl durch chemische Spermizide ersetzt, wobei der Grundgedanke eines Schwammes, welcher mit Spermizid getränkt ist und auf diese Weise empfängnisverhütend wirken soll, beibehalten wurde. Nachteilig bei diesen Einrichtungen ist die umständliche und unangenehme Handhabung des Schwämmchens für seine Positionierung im intravaginalen Bereich. Da das Schwämmchen in seine Position hineingedrückt werden muß, verliert es einen Großteil seiner aufgesaugten Flüssigkeit durch mechanische Kompression und es wurde vor allen Dingen festgestellt, daß sich das feuchte Plastikmaterial nicht angenehm anfühlt.

Eine bekannte Vorrichtung — FR-A-335 672 — beschreibt eine intravaginal positionierbare Vorrichtung mit einem ein Medikament aufnehmenden, schwammförmigen elastischen Tragkörper. Der Tragkörper ist zylinderförmig ausgebildet und weist konzentrisch zur Zylinderachse eine Öffnung auf. Mit dieser Vorrichtung soll es ermöglicht werden, im intravaginalen Bereich ein Medikament einzubringen bzw. eine antiseptische Wirkung zu erzielen. Nachdem eine konzentrisch zur Zylinderachse verlaufende, durchgehende Bohrung angeordnet ist, ist diese Vorrichtung zur Empfängnisverhütung nur bedingt geeignet.

Eine weitere bekannte Vorrichtung — US-A-3 128 762 — beschreibt ebenfalls einen im wesentlichen zylinderförmigen und elastischen aus Kunststoff bestehenden Tragkörper. Der zylinderförmige Tragkörper weist weiters eine konzentrisch zur Zylinderachse verlaufende Öffnung auf. Mit dieser Öffnung soll der Tragkörper auf einen Finger oder einer zur Einführung vorgesehenen Stange positioniert werden, um ihn in die Scheide einzuschieben und an der Portio anzulegen. Dadurch konnte das Positionieren der empfängnisverhütenden Vorrichtung erleichtert werden, schwierig war es jedoch, die eingesetzte Vorrichtung zu entfernen.

Schließlich ist auch eine Vorrichtung zum Manipulieren bekannt — FR-A-2 484 247 — die ein Führungsrohr umfaßt, in welchem in einem Endbereich die empfängnisverhütende Vorrichtung positioniert wird. Vom anderen Endbereich des Führungsrohres her wird eine Schubstange eingeführt, mit der nach dem Einschieben des Führungsrohres in die Scheide die empfängnisverhütende Vorrichtung im Bereich der Portio aus dem Führungsrohr herausgeschoben und im Bereich der Portio positioniert werden kann. Bei dieser Manipuliervorrichtung ist es schwierig, die empfängnisverhütende Vorrichtung im Führungsrohr zu positionieren bzw. wird diese beim Positionieren im Führungsrohr vielfach beschädigt.

Eine weitere bekannte Vorrichtung — US-A-4 219 016 — weist als Tragkörper eine aus mehreren Kunststoffolien bestehende Kappe auf. Diese Kappe weist einen etwa kreisrunden Querschnitt auf und ist in dazu senkrechter Richtung räumlich gewölbt. Im kreisringförmigen Endbereich ist meist ein ringförmig umlaufender Verstärkungsring angeordnet, der diesen Endbereich nach dem Zusammendrücken bzw. Dehnen in die gewünschte Umfangsform zurückführt. Zwischen zwei, insbesondere aus unterschiedlichen Kunststoffen bestehenden Folien ist jeweils ein Medikament oder ein Spermizid angeordnet, mit dem beim Einsatz dieser Vorrichtung als Empfängnisverhütungsmittel die Spermen abgetötet werden sollen. Der labile Aufbau dieser Kappen durch die Verwendung dünner folienartiger elastischer Materialien erschwert sowohl das Einbringen derartiger Tragkörper in die Scheide als auch das Herausnehmen derselben.

Weiters ist es auch bekannt — EP-A-44 102 — derartige kappenartige Tragkörper, insbesondere zur Empfängnisverhütung mittels einer Vorrichtung in die Scheide einzubringen. Das Verfahren zum Einsetzen dieser Kappe ist derart, daß die Kappe in ein U-förmiges Endstück, welches am Ende einer Schubstange angeordnet und mit einer elastischen Membran ausgelegt ist, in zusammengedrücktem Zustand eingeschoben wird. Danach wird der U-förmige Endteil, der über ein Rohr mit einem Blasbalg am anderen Ende der Schubstange verbunden ist, in die Scheide eingeschoben und die Kappe im Bereich der Portio entsprechend positioniert. Dann wird durch Betätigen des Blasbalges die im U-förmigen Halteteil enthaltene, konkav angeordnete Membran in eine konvexe Stellung verbracht und dadurch die Kappe aus dem U-förmigen Endstück ausgestoßen, die sich aufgrund der der kreisringförmigen Wulst der Kappe innewohnenden Elastizität entspannt und um die Portio legt. Damit konnte zwar das Einbringen derartiger Kappen etwas erleichtert werden. Durch die Verwendung von in Einbringrichtung U-förmig geöffneten Organen bzw. Zangen ist jedoch nach wie vor eine Verletzungsgefahr gegeben und überdies ist das Entnehmen derartiger Kappen nur schwierig möglich.

Weitere derartige bekannte Vorrichtungen bestehen aus einem zylinderförmigen Tragkörper aus elastischem Kunststoff bzw. -schwamm, die mit einem Medikament, insbesondere einem Spermizid beschichtet oder getränkt sind. Eine Höhe dieser zylinderförmigen Tragkörper ist geringer als deren Durchmesser. Diese Tragkörper werden von der Benutzerin mit den Fingern in die Scheide eingeschoben und positioniert. Durch die unterschiedliche Lage des Uterus am Ende der Scheide ist dadurch ein eindeutiges Positionieren nur schwer möglich bzw. kann es durch die glatten Stirnseiten des Tragkörpers zu Verlagerungen während eines Geschlechtsverkehrs und damit zu einem Umwirksamwerden der Vorrichtung kommen.

Nach einer weiteren bekannten Vorrichtung — gemäß DE-A-822 877 — ist ebenfalls ein zylinderförmiger Tragkörper vorgesehen, der eine Höhe aufweist, die geringer ist als ein Durchmesser desselben. Eine der Stirnwände des zylinderförmigen Tragkörpers ist konkav gewölbt, wobei diese Wölbung im wesentlichen an den durchschnittlichen Verlauf der Portio des Uterus angepaßt ist. Die gegenüberliegende Stirnwand weist ebenfalls eine konkave Vertiefung auf, oberhalb der ein Gummiband verläuft. Der Tragkörper besteht aus zwei Teilen, die vorzugsweise unter Einschluß einer feuchtigkeitsundurchlässigen Folie miteinander verklebt sind. Der Tragkörper bzw. die einzelnen Teile des Tragkörpers werden durch Natur- oder Kunststoffschwämme gebildet. Der Tragkörper wird vor dem Einbringen in die Scheide mit einem Medikament gefüllt. Nachteilig ist hierbei die mehrteilige Anordnung des Tragkörpers und die damit entstehende Gefahr, daß sich die beiden Teile des Tragkörpers voneinander lösen und dies zu Schwierigkeiten führt. Mit der von der der Portio zugewendeten Seite oberhalb der Öffnung angeordneten Lasche soll das Entnehmen derartiger Vorrichtungen erleichtert werden.

Die Aufgabe der vorliegenden Erfindung liegt darin, eine intravaginal positionierbare, insbesondere empfängnisverhütende Vorrichtung zu schaffen, die eine sichere Positionierung in der Scheide, insbesondere im Bereich der Portio ermöglicht und die einfach eingebracht und entnommen werden kann. Eine weitere Aufgabe der Erfindung liegt darin, eine geeignete Vorrichtung zum Einbringen bzw. Entnehmen derartiger Tragkörper zu schaffen.

Die Aufgabe der Erfindung wird bei einer Vorrichtung der einleitend beschriebenen Art durch die Merkmale im Kennzeichenteil des Patentanspruches 1 gelöst. Der überraschende Vorteil dieser Lösung liegt darin, daß durch den entsprechend ausgebildeten Tragkörper ein guter Sitz desselben in der Scheide, insbesondere im Bereich der Portio erreicht wird und daß außerdem während eines Geschlechtsverkehres der Tragkörper sicher in der gewünschten Lage verbleibt. Außerdem wirkt das Sackloch als Samenreservoir. Auf den im Sackloch gesammelten Samen kann das im Tragkörper enthaltene Medikament intensiv einwirken, wodurch ein Großteil der Spermien sofort und zuverlässig abgetötet werden. Die noch verbleibenden Spermien werden beim Durchtritt durch den Tragkörper durch das in diesem befindliche Medikament zuverlässig abgetötet, so daß bei einer Verwendung als Empfängnisverhütungsmittel ein guter Schutz gegen eine unerwünschte Empfängnis gegeben ist. Ein weiterer Vorteil liegt darin, daß durch die elastische Ausgestaltung des Tragkörpers keine unangenehmen Nebenwirkungen für die Trägerin einer derartigen Vorrichtung auftreten, wobei trotzdem die Elastizität durch eine entsprechende Ausgestaltung des im Inneren angeordneten Mitnehmers sichergestellt werden kann. Weiters wird durch das Verbinden des Tragkörpers und des Mitnehmers über einen Schäumvorgang ein fester Halt des Mitnehmers im Tragkörper erreicht, so daß auch beim Ausüben von größeren Kräften beim Herausziehen der erfindungsgemäßen Vorrichtung ein Ausreißen des Mitnehmers verhindert wird. Dadurch wird auch das Herausnehmen der Vorrichtung erleichtert.

Ein weiteres Merkmal der Erfindung ist im Patentanspruch 2 enthalten. Durch das Einschäumen des Mitnehmers in der beschriebenen Art verbleibt der Mitnehmer in der Öffnung und wird außerhalb derselben, insbesondere in der Scheide nicht als störend empfunden.

Von Vorteil sind weiters auch Ausführungen des Tragkörpers nach den Patentansprüchen 3 und 4. Dadurch wird der Durchtritt von Feuchtigkeit und Körperflüssigkeit möglich, um beispielsweise das im Tragkörper enthaltene Medikament aufzulösen bzw. zu aktivieren, wobei durch die offenen Zellen auch im Bereich der Oberfläche des Tragkörpers sowohl ein Eindringen der Spermien in den Tragkörper als auch ein Austritt des Medikamentes aus dem Tragkörper möglich ist. Durch den gleichbleibenden pH-Wert des offenporigen Kunststoffschaumes weist dieser eine gute Körperverträglichkeit auf.

Weiters ist auch eine Ausbildung nach Patentanspruch 5 möglich. Vorteilhaft ist bei dieser Lösung, daß ein kompakter Tragkörper geschaffen wird, der auch unter der Einwirkung von Körperflüssigkeiten und dgl. bzw. vor allem auch beim Herausziehen aus der Scheide einstückig bleibt und somit ein einfaches Entnehmen sichergestellt ist. Weist der Tragkörper überdies auf der der Öffnung gegenüberliegenden Stirnseite zur Gänze geschlossene Zellen auf — beispielsweise durch die Bildung einer Haut an der Oberfläche des geschäumten Tragkörpers durch entsprechende Temperatursteuerung während des Schäumvorganges, so ist ein Durchtritt von Spermien in Richtung des Uterus gänzlich verhindert.

Vorteilhaft ist auch eine Ausbildung des Tragkörpers nach Patentanspruch 6, da dadurch ein Zerfall in mehrere Einzelteile verhindert wird.

Andere vorteilhafte Ausführungsvarianten sind in den Patentansprüchen 7 und 8 geoffenbart. Vor allem dann, wenn der Übergang einen umlaufenden Randwulst bildet, ist auch bei verschiedenen Lagen des Uterus relativ zur Scheide ein einwandfreies Einsetzen ermöglicht. Vor allem beim Einsetzen und Nachdrücken des Tragkörpers stellt sich der Uterus eher parallel zur Scheide. Nach dem Einsetzen, wenn er in seine ursprüngliche Lage zurückgeht, wird er durch die Portio über den Randwulst mitgenommen und in der entsprechend richtigen Lage gehalten.

Eine andere Ausführungsform ist im Patentanspruch 9 beschrieben. Dadurch wird erreicht, daß auch im Bereich der konkaven Stirnfläche ein durchgehender elastischer Kunststoffschaum vorhanden ist, wodurch eine sichere Einbettung des durch einen Zylinder gebildeten Mitnehmers und ein angenehmes Tragen erreicht wird. Der Kunststoffschaum ist wärmeisolierend und fühlt sich «warm» an, wodurch auch beim Einsetzen des Tragkörpers kein unangenehmes Termperaturverhalten festzustellen ist.

Vorteilhaft sind auch Weiterbildungen nach den Patentansprüchen 10 und 11. Durch die Öffnungen im Zylindermantel werden Laschen gebildet, die mit dem Finger erfaßt bzw. in die ein Stab zum Herausziehen eingehängt werden kann, um ein einfaches und sicheres Herausnehmen der Vorrichtung zu ermöglichen. Dadurch, daß die andere Stirnwand verschlossen ist, kann, wenn der Zylinder aus einem feuchtigkeitsundurchlässigem Material gebildet ist, verhindert werden, daß in dem relativ dünnen Bereich in Richtung der Zylinderachse des Tragkörpers Spermien ungehindert in den Uterus eintreten können. Vielmehr wird der auftreffende Samen zur Seite hin in den umgebenden Bereich des Tragkörpers abgelenkt und kommt dort intensiv mit dem im Tragkörper enthaltenen Spermizid in Verbindung und wird somit abgetötet. Durch die am Zylinder angeordneten Verankerungselemente wird ein sicherer und ausreißfester Sitz des Mitnehmers im Tragkörper sichergestellt. Durch die Anordnung einer durchgehenden Stirnwand im Bodenbereich der sacklochförmigen Öffnung kann sich der Finger bzw. die zum Einbringen verwendete Schubstange auf dieser abstützen, wobei dadurch verhindert wird, daß der dünnwandige Bereich zwischen der Stirnwand des Zylinders und der diesen gegenüberliegenden konkaven Stirnfläche des Tragkörpers beim Einbringen nicht eingerissen bzw. durchgestossen wird, so daß eine zuverlässige Funktion des Tragkörpers sichergestellt ist.

Weiters ist auch eine Ausführung des Mitnehmers nach Patentanspruch 12 möglich. Dies hat den Vorteil, daß die in Richtung der Zylinderachse durchtretenden Spermien zuverlässig quer durch den Tragkörper abgelenkt werden und dort intensiv mit dem im Tragkörper enthaltenen Spermizid in Berührung kommen.

Vorteilhaft ist weiters eine Ausbildung nach Patentanspruch 13. Dadurch, daß derartige Kunststoffe zähelastisch sind und eine hohe Reißfestigkeit aufweisen und gleichzeitig feuchtigkeitsundurchlässig sind, werden die beiden Hauptfunktionen des Mitnehmers, nämlich ein zuverlässiges Einsetzen und Entnehmen der erfindungsgemäßen Vorrichtung, erzielt.

Es ist aber auch eine Ausbildung nach Patentanspruch 14 möglich. Dadurch wird das Einbringen begünstigt und ein Geschlechtsverkehr nicht behindert.

Weiters kann ein Tragkörper auch gemäß Patentanspruch 15 ausgebildet sein, wodurch eine gute Elastizität in radialer Richtung erreicht wird.

Von Vorteil ist auch eine Ausbildung nach Patentanspruch 16, da dadurch ein ausreichendes Volumen des Tragkörpers zur Aufnahme des Spermizides zur Verfügung steht.

Eine andere Ausführungsvariante ist nach Patentanspruch 17 vorgesehen. Dadurch wird der Vorteil erreicht, daß der Tragkörper durch den eingearbeiteten Netzkörper verstärkt wird und überdies das Herausnehmen durch das Einhängen in eine Netzöffnung aber trotzdem leicht möglich ist.

Vorteilhaft ist auch eine Weiterbildung des Mitnehmers nach den Patentansprüchen 18 und 19, wodurch das Einbringen der Vorrichtung auch mit dem Finger und vor allem aber das Herausziehen der Vorrichtung mit dem Finger erleichtert wird.

Es ist aber auch eine Ausbildung nach Patentanspruch 20 möglich, wodurch das Durchdringen von Sperma noch zusätzlich erschwert wird.

Auch ist eine Ausbildung nach Patentanspruch 21 möglich, wodurch das Entstehen von gesundheitsschädlichen Substanzen durch Ausdampfen von Lösungsmitteln oder dgl. zuverlässig vermieden ist.

Von Vorteil ist aber auch eine Weiterbildung nach Patentanspruch 22, wodurch eine bessere Haftung zwischen diesen beiden Teilen und eine bessere Stabilität der Vorrichtung erreicht wird.

Eine andere Ausführungsvariante ist im Patentanspruch 23 vorgesehen, wodurch die spermizide Aktivität der Vorrichtung verbessert wird.

Vorteilhaft ist aber auch eine Ausführung nach Patentanspruch 24. Auf diese Weise wird eine Ausbildung geschaffen, bei welcher die Klebestellen und damit eine theoretisch mögliche Verhärtung auf den Umfang der Vorrichtung bzw. des Schwammes beschränkt sind, wohingegen der mittlere Teil der im wesentlichen dichten Folie nach Art einer Membran am Tragkörper anliegt. Die Entfernung einer derartigen Vorrichtung bzw. eines derartigen Schwämmchens könnte beispielsweise unter Anwendung eines Unterdruckes erfolgen, wobei sich dann diese Membran in einen entsprechenden Saugschlauch einsaugen läßt.

Weiters ist aber auch eine Ausbildung nach Patentanspruch 25 möglich, wodurch das Positionieren und Entfernen des Schwämmchens erleichtert wird.

Von Vorteil ist dabei eine Ausführung nach Patentanspruch 26, da eine Verankerung eines Greifers zum Entfernen der Vorrichtung vereinfacht wird.

Schließlich umfaßt die Erfindung auch eine Manipuliervorrichtung mit einer Schubstange zum intravaginalen Positionieren der Vorrichtung.

Diese Manipuliervorrichtung ist im Patentanspruch 27 gekennzeichnet. Durch die Verwendung eines pilzförmigen Ansatzes wird die Kraft beim Zusammendrücken des Tragkörpers im Trichter und beim Durchschieben durch das Führungsrohr gleichmäßig in den Tragkörper eingeleitet und es wird ein Durchreißen bzw. ein Zerstören des Tragkörpers vermieden. Durch den sich unmittelbar an den pilzförmigen Ansatz anschließenden verjüngten Bereich kann das Material des Tragkörpers ohne zu starke Quetschung zwischen der Oberfläche der Schubstange und der inneren Oberfläche des Führungsrohres positioniert werden. Weiters wird es durch die konkave Ausgestaltung des der Gebärmutter zugeordneten Endes des Führungsrohres möglich, eine starke Abrundung des Führungsrohres vorzunehmen, wodurch Verletzungen beim Einschieben des Führungsrohres in die Scheide vermieden werden.

Vorteilhaft ist eine weitere Ausbildung nach Patentanspruch 28, da dadurch das Herausziehen der Schubstange nach dem Einsetzen der Vorrichtung in der Scheide nicht behindert wird.

Eine andere Ausführungsvariante beschreibt Patentanspruch 29. Dadurch kann der Tragkörper im Aufnahmebereich des Trichters einfach positioniert werden und es erfolgt ein kontinuierliches Zusammendrücken desselben während des Einschieben in Richtung des Führungsrohres.

Von Vorteil ist auch eine Weiterbildung nach Patentanspruch 30, wodurch das Herausnehmen der Vorrichtung mit der Schubstange erleichtert wird.

Vorteilhaft ist aber auch eine weitere Ausbildung nach Patentanspruch 31, da durch die Verwendung des Greifers die Vorrichtung bzw. der Schwamm erfaßt und damit das Positionieren und Entfernen erleichtert wird.

Schließlich ist auch eine Weiterbildung nach Patentanspruch 32 möglich. Dadurch wird auch bei einer Ausführungsform, bei welcher die Vorrichtung bzw. das Schwämmchen durch Klauen erfaßt wird, eine Verletzungsgefahr beim Einsetzen und Entfernen der Vorrichtung bzw. des Schwämmchens hintangehalten.

Zum besseren Verständnis der Erfindung wird diese im folgenden anhand der in den Zeichnungen dargestellten Ausführungsbeispiele näher erläutert.

Es zeigen:

Fig. 1 eine erfindungsgemäße Vorrichtung in Seitenansicht mit geschnittenem Tragkörper und Mitnehmer;

Fig. 2 eine Draufsicht auf die Vorrichtung nach Fig. 1;

Fig. 3 eine Ausführungsvariante eines Mitnehmers in Seitenansicht;

Fig. 4 eine erfindungsgemäß ausgebildete Manipuliervorrichtung **während des Durchschiebens** des Tragkörpers **einer erfindungsgemäßen Vorrichtung** durch das Führungsrohr in Seitenansicht und teilweise geschnitten;

Fig. 5 eine andere Ausführungsform einer Schubstange der Manipuliervorrichtung beim Herausziehen einer erfindungsgemäßen Vorrichtung aus der Scheide;

Fig. 6 eine schematische Darstellung der in der Scheide positionierten Manipuliervorrichtung während des Einschiebens der erfindungsgemäßen Vorrichtung in die Scheide;

Fig. 7 eine schematische Darstellung nach dem Positionieren des Tragkörpers der erfindungsgemäßen Vorrichtung im Bereich der Portio des Uterus;

Fig. 8 eine schematische Darstellung des Uterus und der Scheide während des Herausziehens einer erfindungsgemäßen Vorrichtung mit der Schubstange;

Fig. 9 eine erfindungsgemäße Vorrichtung während des Herausziehens aus der Scheide mit dem Finger.

Fig. 10 eine andere Ausführung einer erfindungsgemäßen Vorrichtung axial geschnitten;

Fig. 11 einen weiten Schnitt durch eine anders ausgebildete Vorrichtung axial geschnitten;

Fig. 12 eine Manipuliervorrichtung im Axialschnitt in derjenigen Zusammenstellung, in welcher die Entfernung einer erfindungsgemäßen Vorrichtung vorbereitet wird;

Fig. 13 die Manipuliervorrichtung nach Fig. 3 in Stirnansicht;

Fig. 14 den Applikator nach Fig. 12 in der Eingriffsstellung für das Entfernen einer Vorrichtung nach Fig. 11;

Fig. 15 eine Stirnansicht auf den die Manipuliervorrichtung in Richtung des Pfeiles X in Fig. 14 bei abgenommener Vorrichtung;

Fig. 16 eine in einer Verpackung komprimierte gehaltene antikonzeptive Vorrichtung im Axialschnitt;

Fig. 17 die Anordnung derselben in der Manipuliervorrichtung zum Positionieren in der Scheide.

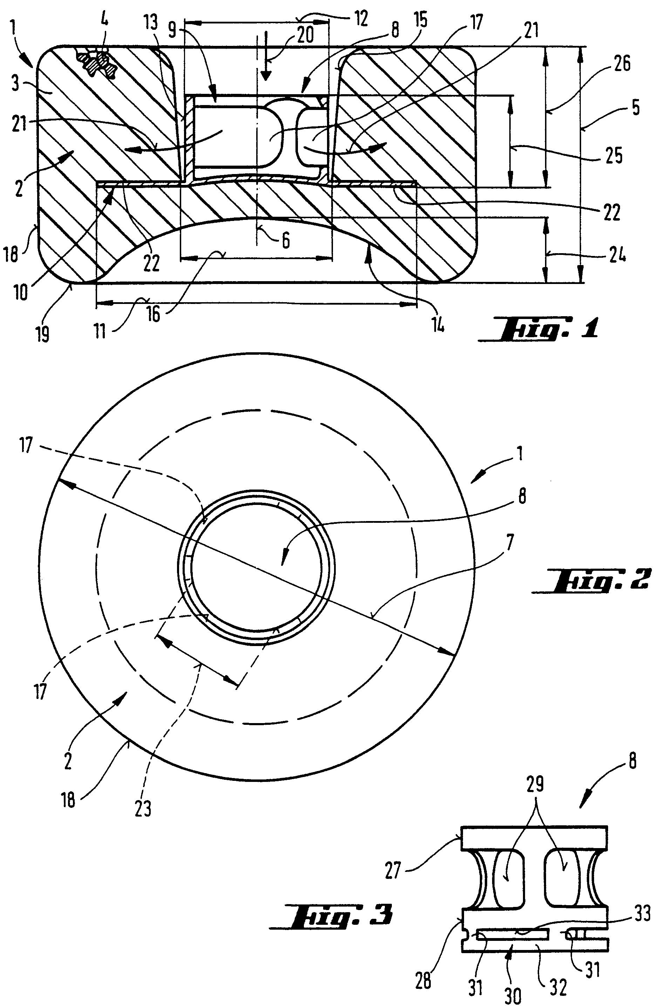

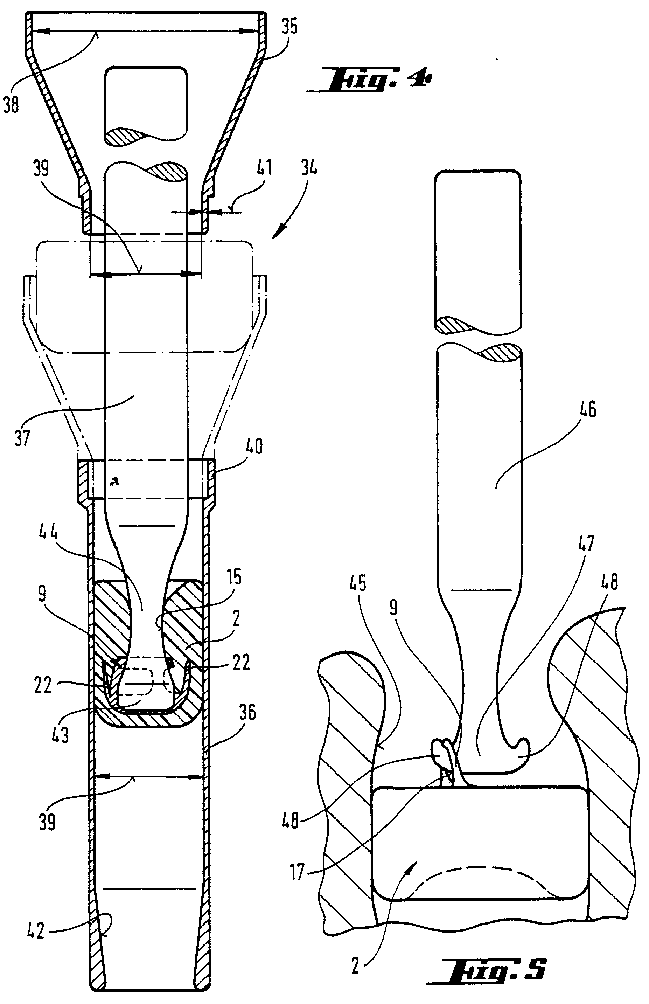

In Figur 1 und 2 ist eine intravaginal positionierbare Vorrichtung 1 dargestellt. Diese Vorrichtung 1 besteht aus einem Tragkörper 2, der durch einen zylinderförmigen Block aus einem Kunststoffschaum 3, z.B. einem Polyurethanschaum hergestellt ist. Dieser Kunststoffschaum weist zum großen Teil offene Zellen auf, wie dies im Schnittbereich schematisch angedeutet ist. Diese offenen Zellen sind, wie durch Schraffur angedeutet, mit einem Medikament 4, welches in den fertiggestellten Kunststoffschaum 3 eingespritzt werden kann, enthalten.

Der Tragkörper 2 weist, wie am besten aus Figur 2 ersichtlich ist, einen kreisrunden Querschnitt auf. Eine Höhe 5 parallel zu einer Zylinderachse 6 des Tragkörpers 2 ist kleiner als ein äußerer Durchmesser 7 des Tragkörpers, wodurch eine gute Elastizität in radialer Richtung des Durchmessers erreicht wird.

In dem Tragkörper ist während des Schäumvorganges zur Herstellung desselben ein Mitnehmer 8 eingeschäumt. Im vorliegenden Ausführungsbeispiel wird der Mitnehmer 8 durch einen Zylinder 9 gebildet, der im Bereich einer seiner beiden Stirnseiten durch eine Stirnwand 10 verschlossen ist. Diese Stirnwand 10 weist einen Durchmesser 11 auf, der größer ist als ein äußerer Durchmesser 12 des Zylinders 9. Mit dem über den äußeren Durchmesser bzw. einen Zylindermantel 13 vorspringenden Bereich der Stirnwand 10 ist diese im Tragkörper 2 verankert. Wie ersichtlich, bildet diese Stirnwand 10 den Abschluß einer gegen eine Stirnfläche 14 gerichteten durch ein Sackloch gebildeten Öffnung 15. Diese parallel zur Zylinderachse 6 verlaufende Öffnung 15 ist im vorliegenden Fall konisch ausgebildet und weist im Bereich der Stirnwand 10 des Mitnehmers 8 einen Durchmesser 16 auf, der größer ist als der äußere Durchmesser 12 des Zylinders 9. Damit steht der Zylindermantel 13 innerhalb der Öffnung 15 völlig frei. Da im Zylindermantel 13 überdies Öffnungen 17 angeordnet sind, bildet dieser Zylindermantel 13 mehrere in Umfangsrichtung aneinanderschließende Schlaufen bzw. Laschen, in die beim Herausnehmen der Vorrichtung aus der Scheide ein Entnahmeorgan bzw. ein Finger eingehängt werden kann, um den Tragkörper 2 herauszuziehen.

Selbstverständlich ist es aber auch möglich, die Öffnung 15 mit einem kreisrunden Querschnitt auszubilden und den Durchmesser 16 so zu wählen, daß er im wesentlichen dem äußeren Durchmesser 12 des Zylinders 9 entspricht.

Die Stirnfläche 14 des Tragkörpers 2 ist konkav ausgebildet, wobei der Übergang zwischen dieser konkaven Stirnfläche und einer Mantelfläche 18 stark abgerundet ist und insbesondere einen umlaufenden Randwulst 19 bildet. Die vom Randwulst umschlossene Fläche ist in ihrer Gestaltung der durchschnittlichen Ausbildung der Portio, also dem Gebärmuttereingang angepaßt, so daß sich ein guter Verschluß der Gebärmutter ergibt. Dies hat den Vorteil, daß, falls der Tragkörper 2 mit zur Behandlung der Scheide oder der Gebärmutter dienenden Medikamenten gefüllt ist, diese in die entsprechenden Bereiche abgesetzt werden können und daß dann, wenn die Vorrichtung 1 als Empfängnisverhütungsmittel verwendet wird, eine satte Anlage und ein guter Abschluß der Portio gegeben ist.

Im Falle der Verwendung der Vorrichtung 1 als empfängnisverhütendes Mittel ist es von Vorteil, wenn die Stirnwand 10 einen Durchmesser 11 aufweist und aus einem flüssigkeitsundurchlässigem Kunststoff, beispielsweise einem elastischen Silikonkautschuk und bzw. oder Weich-PVC und bzw. oder einem Pharmagummi und bzw. oder thermoplastischem Kautschuk, wie beispielsweise Kraton besteht. Die parallel zur Zylinderachse 6 in Richtung eines Pfeiles 20 während eines Geschlechtsverkehres eindringenden Spermien werden durch diese undurchlässige Stirnwand 10, wie schematisch durch Pfeile 21 angedeutet, in den Tragkörper 2 abgelenkt und kommen dort beim Durchtritt durch die offenen Zellen in intensive Berührung mit dem darin enthaltenen Medikament 4 oder Spermizid. Dadurch werden die Spermien abgetötet. Überdies ist der Durchgangsquerschnitt zwischen der Mantelfläche 18 des Tragkörpers 2 und der Stirnwand 10 so eingeengt, daß ein schneller Durchtritt von Spermien oder Schadstoffen durch den Tragkörper nahezu ausgeschlossen ist. Vor allem wird verhindert, daß in dem Bereich mit dünner Wandstärke zwischen der Stirnwand 10 und der Stirnfläche 14 Spermien oder Schadstoffe rasch durch die Richtung der Gebärmutter durchtreten können und es wird durch diese Stirnwand 10 zusätzlich auch das Ausreißen der Kunststoffschaumes während des Einbringens oder während des Geschlechtsverkehres ausgeschalten.

Trotzdem wird aber mit Vorteil erreicht, daß keine über den Tragkörper 2 heraushängende oder vorstehende Teile als Mitnehmer zum Herausziehen oder Einbringen des Tragkörpers 2 in der Scheide vorhanden sind.

Die über den äußeren Durchmesser 12 vorspringenden Teile der Stirnwand 10 bilden Verankerungselemente 22, die die sichere Halterung des Mitnehmers 8 im Kunststoffschaum 3 des Tragkörpers 2 ermöglichen.

Wie weiters schematisch besser aus Figur 2 zu ersehen ist, weisen die Öffnungen 17 einen Durchmesser 23 auf, der im wesentlichen einem Durchmesser eines menschlichen Fingers entspricht, so daß die durch den Zylindermantel 13 gebildeten Laschen leicht erfaßt und zum sicheren Herausziehen des Tragkörpers 2 aus der Scheide verwendet werden können.

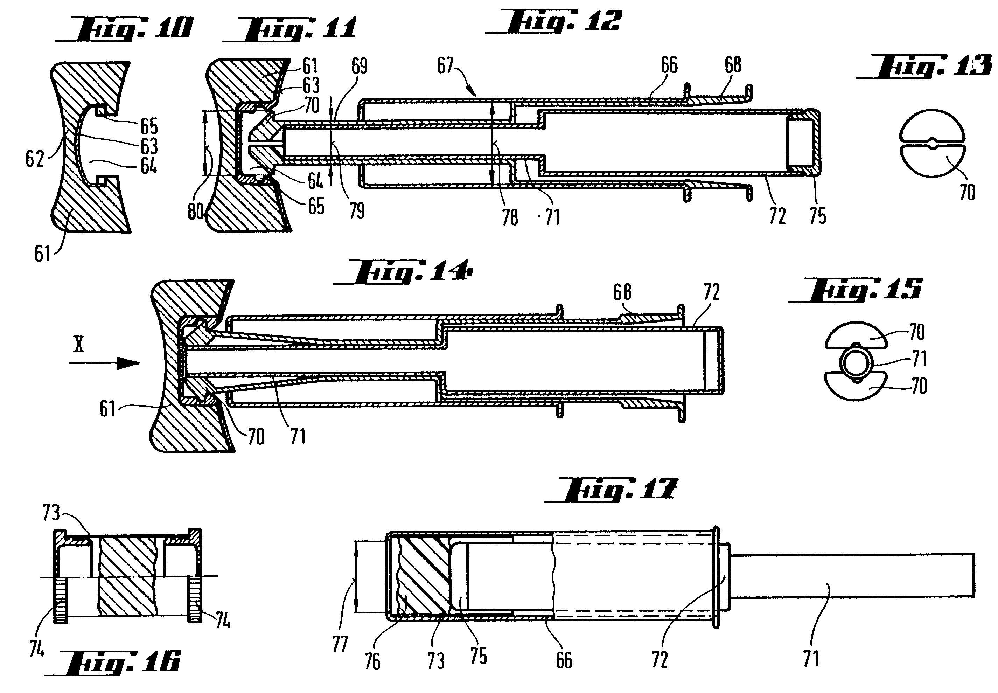

Die entsprechende Gestaltung der Stirnfläche 14 in Anpassung an den Verlauf der Portio am Eingang der Gebärmutter verhindert überdies — wie dies im folgenden anhand der schematischen Darstellungen in den Figuren 6 bis 9 noch erläutert werden wird — bei unterschiedlichen Stellungen des Uterus, insbesondere bei anti vertiert flektiertem Uterus der Tragkörper nach dem Positionieren an der Portio durch das Zurückstellen des Uterus in die natürliche Lage beim Herausziehen der Finger bzw. des Führungsrohres aus der Scheide von der Portio abrutscht und keinen Schutz mehr gewährt.

Um das Durchdringen von Schadstoffen in den Uterus zu unterbinden, ist es auch möglich, die Stirnfläche 14 mit geschlossenen Poren auszubilden, wobei dies bei Verwendung von Polyurethan-Kunststoffschaum derart erfolgen kann, daß durch eine entsprechende Temperatursteuerung in der Form, in dem dieser Bereich der Form kühler gehalten wird als die übrigen Bereiche, während des Schäumvorganges eine dichte Haut gebildet wird, so daß eine dichte Stirnfläche 14 erzielt wird.

Von Vorteil ist es weiters, wenn der Durchmesser 12 des Zylinders 9 kleiner ist als der halbe Durchmesser 7 des Tragkörpers 2. Dadurch verbleibt genügend Volumen an offenporigem Kunststoffschaum zur Aufnahme einer entsprechenden Menge eines Medikamentes.

Eine Tiefe 24 der konkaven Wölbung der Stirnfläche 14 ist geringer als die Höhe 5 des Tragkörpers 2 abzüglich einer Gesamthöhe 25 des den Mitnehmer 8 bildenden Zylinders 9.

Im vorliegenden Ausführungsbeispiel ist eine Sacklochtiefe 26 der Öffnung 15 größer als die Gesamthöhe 25 des Mitnehmers 8. Es ist aber selbstverständlich im Rahmen der Erfindung auch möglich, daß die Sacklochtiefe 26 und die Gesamthöhe 25 in etwa gleich groß sind.

In Figur 3 ist eine Ausführungsvariante des Mitnehmers 8 dargestellt. Dieser Mitnehmer 8 besteht aus einem Zylinder 27, in dessen Zylindermantel 28 Öffnungen 29 — die den Öffnungen 17 des Mitnehmers 8 in Fig. 1 und 2 entsprechen — enthalten sind. Als Verankerungselement 30 ist ein über Stege 31 distanzierter Ring 32 aus Kunststoff angeordnet. Durch diesen von einer Stirnwand 33 des Zylinders 27 distanzierten Ring 32 wird eine Verankerung des Mitnehmers 8 im Kunststoffschaum 3 des Tragkörpers 2 erreicht.

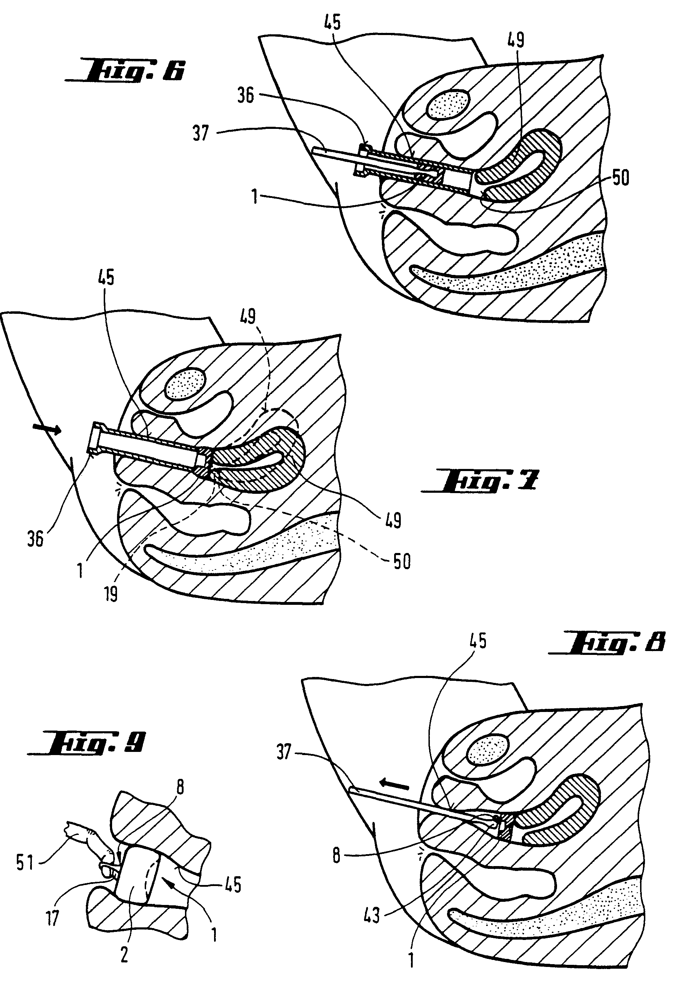

In Figur 4 ist eine Manipuliervorrichtung 34 gezeigt, die aus einem Trichter 35 und einem Führungsrohr 36 sowie einer Schubstange 37 besteht. Der Trichter 35 weist einen zylinderförmigen Einlaß auf, der einen Durchmesser 38 aufweist, der im wesentlichen einem Durchmesser 7 des Tragkörpers 2 entspricht. An diesen schließt ein konischer Bereich an, der in einen zylinderförmigen Übergangsteil mündet, der einen inneren Durchmesser 39 aufweist, der identisch mit einem inneren Durchmesser 39 des Führungsrohres 36 ist. Das Führungsrohr 36 weist einen Flansch 40 auf, dessen zylinderförmige Innenfläche einen um die doppelte Wandstärke 41 des Trichters 35 größeren Durchmesser aufweist als der innere Durchmesser 39 des Führungsrohres 36. Das Führungsrohr 36 weist in seinem dem Flansch 40 gegenüberliegenden Endbereich eine konische Fläche 42 auf, die über eine starke Abrundung mit der Außenfläche des Führungsrohres 36 verbunden ist. Die Schubstange 37 wird zum Einsetzen einer Vorrichtung 1 mit dem Tragkörper 2 in die Öffnung 15 bzw. in die Innenseite des den Mitnehmer 8 bildenden Zylinders 9 mit einem pilzförmig ausgebildeten Ansatz 43 eingeschoben — strichlierte Darstellung im Einlaßbereich des Trichters —. Dann wird durch das Vorwärtsbewegen der Schubstange 37 in Richtung des Führungsrohres 36 der Tragkörper 2 zusammengepreßt und in die Länge gezogen, sowie dies aus der in vollen Linien im Anfangsbereich des Führungsrohres gezeigten Darstellung ersichtlich ist. Durch einen verjüngten Bereich 44 der Schubstange 37 unmittelbar anschließend an den pilzförmigen Ansatz 43 kann der Zylinder 9 un der Tragkörper 2 nach innen zusammengedrückt werden, so daß ein leichteres Durchschieben des Tragkörpers 2 durch das Führungsrohr 36 ermöglicht wird. Die Verankerungselemente 22 und der Zylinder 9 werden durch das Zusammendrücken vorgespannt und wirken beim Austreten des Tragkörpers 2 am Ende des Führungsrohres 36 in Art einer in radialer Richtung des Tragkörpers 2 wirkenden Springfeder, um diesen Tragkörper in seine endgültig positionierte Lage im Bereich der Portio.

In Figur 5 ist ein Tragkörper 2 im Bereich des Ausganges einer Scheide 45 gezeigt, wobei eine Schubstange 46, die im Bereich ihres pilzförmigen Ansatzes 47 mit Mitnehmer-Noppen 48 versehen ist, in eine Öffnung 17 des Zylinders 9 eingehängt ist, um den Tragkörper 2 aus der Scheide 45 herauszuziehen.

In den Figuren 6 bis 8 ist schematisch die Scheide 45 mit einer daran anschließenden Gebärmutter und dem Gebärmuttereingang der Portio 50 schematisch dargestellt.

Anhand dieser Darstellungen soll das Verfahren zum Einbringen bzw. Einsetzen und Entnehmen bzw. Herausziehen der erfindungsgemäßen Vorrichtung mit dem Tragkörper und unter Verwendung des Mitnehmers 8 erläutert werden.

Wie bereits anhand der Darstellung in Figur 4 beschrieben, wird die Vorrichtung 1 durch Zusammenpressen des Tragkörpers 2 und des Mitnehmers 8 im Trichter 35 im Führungsrohr 36 positioniert. Dann wird der Trichter 35 abgezogen und das Führungsrohr 36 in der Scheide 45 — Figur 6 — positioniert und zwar so weit eingeschoben, bis es nahezu am Ende der Scheide ansteht. Danach wird mit der Schubstange 37 die Vorrichtung 1 so weit relativ zum Führungsrohr 36 in Richtung der Gebärmutter 49 verschoben, bis der Tragkörper 2 aus dem Führungsrohr 36 austritt und sich durch die diesen innewohnende Elastizität des Kunststoffschaumes des Tragkörpers 2 sowie die Federwirkung der Verankerungselemente 22 und des Mitnehmers 8 in seine volle Querschnittsgröße ausdehnt. Mit dem Führungsrohr 36 wird die ausgedehnte in die volle Größe expandierte Vorrichtung 1 festgehalten, während die Schubstange 37 aus dem Führungsrohr 36 herausgezogen wird. Durch Nachschieben mit dem Führungsrohr 36 kann, wie in Figur 7 gezeigt, die Vorrichtung 1 nunmehr vor der Portio 50 der Gebärmutter exakt positioniert werden. Dabei ist festzuhalten, daß, wie dies ebenfalls stark übertrieben schematisch angedeutet ist, auch bei unterschiedlichen Lagen der Gebärmutter 49, beispielsweise vor allem bei anti vertiert flektierten Lagen durch das Einschieben des Führungsrohres 36 diese in die in vollen Linien gezeichnete Lage verstellt wird, so daß das Positionieren der Vorrichtung 1 exakt erfolgen kann. Wird dann das Führungsrohr 36 aus der Scheide 45 entnommen, weicht die Gebärmutter 49 in ihre ursprüngliche — mit strichlierten Linien gezeichnete Lage — zurück. Durch die Randwulst 19 wird die Vorrichtung 1 jedoch durch die Portio 50 mitgenommen und schließt auch in der veränderten Lage der Gebärmutter 49 die Portio zuverlässig ab.

Das Entnehmen der Vorrichtung 1 aus der Scheide 45 ist aus Figur 8 besser ersichtlich. Die Schubstange 37 wird mit ihrem pilzförmigen Ansatz 43 bzw. 47 in den Mitnehmer 8 eingehängt und durch eine Bewegung in Richtung des Pfeiles aus der Scheide 45 herausgezogen.

In Figur 9 ist gezeigt, daß die Benutzerin auch mit dem Finger 51 in Öffnungen 17 bzw. 29 des Mitnehmers 8 hineingreifen kann, um die Vorrichtung 1 bzw. den Tragkörper 2 aus der Scheide 45 herauszuziehen.

Die Teile der Manipulationsvorrichtung werden vorteilhafter Weise aus durchsichtigen Plexiglas gefertigt, so daß sie eine möglichst glatte Oberfläche aufweisen und die Reinigung einfach möglich ist bzw. die Reinheit der einzelnen Teile durch die Benutzerin leicht geprüft werden kann. Außerdem weist dieses Material eine entsprechende Festigkeit auf, um die notwendigen Manipulationsvorgänge zum Einsetzen der Vorrichtungen 1 zu ermöglichen.

Wird das Medikament im flüssigen oder zumindest im zähflüssigen Zustand in den Tragkörper 2 injiziert bzw. eingebracht, so wirkt es beim Durchschieben durch das Führungsrohr 36 wie ein Schmiermittel, wodurch das Durchschieben noch zusätzlich erleichtert wird.

Ein weiterer Vorteil der Ausbildung des Führungsrohres 36 und der Schubstange 37 liegt darin, daß keine kantigen oder vorspringenden bzw. zangenartig beweglichen Teile erforderlich sind und somit eine Verletzungsgefahr im Bereich der Scheide bzw. der Gebärmutter verhindert ist.

Selbstverständlich ist es im Rahmen der Erfindung möglich, den Tragkörper 2 aus den unterschiedlichsten Materialien herzustellen, die unter den Bedingungen im Bereich der Scheide bzw. Gebärmutter, d.h. bei Körpertemperatur einen pH-Wert von ca. 4 über längere Zeit ihren Zustand aufrecht erhalten und keine Giftstoffe oder schleimhautschädliche Lösungen oder dgl. abgeben.

Gleichermaßen können auch für die Mitnehmer unterschiedliche Materialien verwendet werden, wobei sich vor allem das Material Kraton, also ein thermoplastischer Kautschuk besonders bewährt hat. Für die Herstellung des Tragkörpers 2 eignen sich vor allem Polyurethan-Weichschäume mit einem sehr niederen Raumgewicht und einer offenzelligen Struktur.

Selbstverständlich ist es auch möglich, die Außenform der Vorrichtung 1 insbesondere im Bereich der Stirnfläche oder auch im Bereich der Umfangsfläche des Zylindermantels oder der der Stirnfläche gegenüberliegenden Stirnseite beliebig auszubilden. Darüberhinaus muß auch die Querschnittsform in Einbringrichtung nicht kreisrund sondern kann auch elliptisch oder unter Umständen mehreckig oder dgl. sein.

In Fig. 10 ist eine antikonzeptive Vorrichtung bzw. ein Schwämmchen dargestellt, dessen Grund- bzw. Tragkörper 61 aus porösem saugfähigem Material besteht. Eine freie Stirnfläche 62 dieses Grundkörpers ist konkav ausgebildet und für die Positionierung an der Portio vorgesehen. Eine dieser Stirnfläche gegenüberliegende Stirnfläche ist von einer Folie 63 aus elastomerem Material abgedeckt, wobei diese Folie vollflächig mit dem Tragkörper 61 verklebt ist. Die Folie 63 kleidet hiebei eine Ausnehmung bzw. Öffnung 64 aus, welche nach innen vorspringende Ränder 65 für den Eingriff eines Applikators bzw. einer Manipuliervorrichtung aufweisen.

Bei einer Ausbildung nach Fig. 11 ist die Ausnehmung bzw. Öffnung 64 mit einer in Umfangsrichtung verlaufenden Nut 65 versehen, welche gleichfalls für den Eingriff einer Manipuliervorrichtung gedacht ist. Auch hier ist die außen liegende Stirnfläche mit einer Folie 63 aus elastomerem Material abgedeckt, welche ganzflächig mit dem Tragkörper 61 verklebt ist.

In der Darstellung nach Figur 12 ist ein rohrförmiger Führungsteil 66 eines Applikators bzw. einer Manipuliervorrichtung 67 dargestellt, wobei in der Zusammenstellung nach Figur 12 der Applikator für die Entfernung einer Vorrichtung bzw. eines Schwämmchens nach Figur 11 zusammengesetzt ist. Zu diesem Zweck ist in den rohrförmigen Führungsteil 66 ein zweiter rohrförmiger Teil 68 eingeschoben, dessen Außendurchmesser kleiner ist als der Innendurchmesser des rohrförmigen Führungsteiles 66. Dieser zweite rohrförmige Teil 68 trägt einen rohrförmigen Ansatz 69, an dessen freien Enden nach außen verformbare Klauen 70 angeordnet sind. Der Innendurchmesser dieses rohrförmigen Ansatzes 69 ist wiederum größer als der Außendurchmesser eines Fortsatzes 71 eines in die Anordnung eingeschobenen Schiebers 72. Dieser Fortsatz 71 kann in das Innere des rohrförmigen Ansatzes 69 eingeschoben werden. Der rohrförmige Ansatz 69 verjüngt sich im Inneren im Bereich der Klauen konisch, so daß das Einschieben des Fortsatzes 71 eine Aufweitung der Klauen 70 zur Folge hat. Dies ist in Figur 14 deutlich dargestellt, wobei hier der Schieber 72 vollkommen eingeschoben dargestellt ist. Die Klauen 70 greifen hiebei in die Nut 65 des Tragkörpers bzw. Schwammes ein und erlauben das Entfernen desselben. Die nicht aufgeweitete Position der Klauen ist hiebei aus der Stirnansicht in Figur 13 ersichtlich, wohingegen die aufgeweitete Stellung, wie sie für den Eingriff in die Nut 65 gemäß der Darstellung nach Figur 14 vorgesehen ist, aus der Stirnansicht in Figur 15 ersichtlich ist.

Die gleiche Einrichtung wie nach den Figuren 12 bis 15 kann nach Entfernen des zweiten rohrförmigen Teiles 68 in einfacher Weise auch für das Positionieren des Tragkörpers bzw. Schwämmchens verwendet werden. Zu diesem Zweck ist, wie in Figur 16 dargestellt, das Schwämmchen in einer Einwegverpackung enthalten, in welcher der Tragkörper bzw. das Schwämmchen in radialer Richtung komprimiert enthalten ist. Die Verpackung besteht aus einer Hülse 73, welche nach Entfernen der beiden Verschlußkappen 74 in das Innere des rohrförmigen Führungsteiles 66 eingelegt werden kann. Der Schieber 72 weist, wie in Figur 12 ersichtlich, an seinem freien Ende ein Verschlußteil 75 auf und kann nun mit diesem Verschlußteil als Stirnfläche in das Innere des rohrförmigen Führungsteiles 66 eingeführt werden. Der Fortsatz 71 bietet einen Betätigungsangriff und durch Einschieben des Schiebers 72 wird der komprimierte Schwamm bzw. die Vorrichtung 76 aus seiner Verpackungshülse 73 ausgestoßen und paßt sich nach Expansion in radialer Richtung dem Anbringungsort an.

Wie am besten aus Figur 17 zu ersehen ist, weist der rohrförmige Führungsteil 66 in seinem zum Aufnehmen der Vorrichtung 76 ausgebildeten Endbereich einen geringeren Durchmesser 77 auf als ein Innendurchmesser 78 dieses Führungsteiles. Ein Außendurchmesser 79 des rohrförmigen Ansatzes 69 ist ebenso wie ein Außendurchmesser 80 der Klauen 70 im entspannten Zustand geringer als der Durchmesser 77. Durch diese Durchmesserverengung des rohrförmigen Führungsteiles 66 kann die Hülse 73 der Verpackung für die Vorrichtung während des Ausschiebens derselben mit dem Verschlußteil 75 — siehe Figur 17 — einfach zurückgehalten werden.

Das Spreizen der Klauen 70 erfolgt bei der Manipuliervorrichtung derart, daß beim Vorschieben des Fortsatzes 71 in Richtung der Klauen 70 der rohrförmige Teil 68 sich an dem Führungsteil 66 abstützt und durch die Relativbewegung zwischen dem Fortsatz 71 und dem rohrförmigen Ansatz 69 über die konischen Flächen auf der Innenseite der Klauen diese in die in Figur 14 gezeigte Stellung auseinander gespreizt werden. Dadurch, daß der Fortsatz 71 und der Ansatz 69 im wesentlichen eine gleiche Länge aufweisen, wird verhindert, daß der Fortsatz 71 über die Klauen 70 vorgeschoben und der Tragkörper 61 bzw. die Vorrichtung 76 von den Klauen 70 heruntergedrückt wird.

Selbstverständlich ist es auch bei dieser Ausführungsform der Manipuliervorrichtung möglich, die Vorrichtung 76 durch die Verwendung eines Trichters zu komprimieren und in das Innere des rohrförmigen Führungsteiles 66 einzubringen. Im letzt genannten Fall weist der rohrförmige Teil 66 dann einen gleichbleibenden Innendurchmesser 78 auf.

**Patentansprüche**

1. Intravaginal positionierbare, insbesondere empfängnisverhütende Vorrichtung (1) mit einem ein Medikament (4) aufnehmenden, aus einem im wesentlichen zylinderförmigen Block aus Kunststoff bestehenden, elastischen Tragkörper (2), der mit einer konzentrisch zur Zylinderachse (6) verlaufenden Öffnung (15) versehen ist, dadurch gekennzeichnet, daß der Tragkörper (2) aus Kunststoffschaum (3) gebildet ist und die Öffnung (15) sacklochförmig ausgebildet ist und in dieser ein Mitnehmer (8) angeordnet ist, der mit dem Tragkörper (2) durch einen Schäumvorgang verbunden ist.

2. Vorrichtung nach Anspruch 1, dadurch gekennzeichnet, daß der Mitnehmer (8) mit einer dem Tragkörper (2) zugewandten Oberfläche, in die sacklochförmige Öffnung (15) eingeschäumt ist.

3. Vorrichtung nach Anspruch 1 oder 2, dadurch gekennzeichnet, daß der Tragkörper (2) aus einem offenporigen Kunststoffschaum (3) besteht, der vorzugsweise auch im Bereich der Oberfläche offene Zellen aufweist.

4. Vorrichtung nach Anspruch 3, dadurch gekennzeichnet, daß der offenporige Kunststoffschaum (3) ein Polyurethan-Weichschaum ist.

5. Vorrichtung nach einem der Ansprüche 1 bis 4, dadurch gekennzeichnet, daß auf der der sacklochförmigen Öffnung (15) gegenüberliegenden Seite insbesondere durch entsprechende Temperatursteuerung während des Schäumvorganges die Zellen geschlossen sind.

6. Vorrichtung nach einem der Ansprüche 1 bis 5, dadurch gekennzeichnet, daß der Tragkörper (2) einteilig ausgebildet ist.

7. Vorrichtung nach einem der Ansprüche 1 bis 6, dadurch gekennzeichnet, daß die der Öffnung (15) gegenüberliegende Stirnfläche (14) des Tragkörpers (2) konkav ausgebildet ist und der Übergang zwischen dieser konkaven Stirnfläche (14) und einer Mantelfläche (18) des Tragkörpers (2) abgerundet ist.

8. Vorrichtung nach Anspruch 7, dadurch gekennzeichnet, daß der Übergang zwischen Stirn- und Mantelfläche (14, 18) einen umlaufenden Randwulst (19) bildet.

9. Vorrichtung nach einem der Ansprüche 1 bis 8, dadurch gkennzeichnet, daß die Tiefe (24) dier Wölbung der konkaven Stirnfläche (14) geringer ist als die Höhe (5) des Tragkörpers (2) abzüglich der Gesamthöhe (25) des den Mitnehmer (8) bildenden Zylinders (9).

10. Vorrichtung nach einem der Ansprüche 1 bis 9, dadurch gekennzeichnet, daß der Mitnehmer (8) aus einem Zylinder (9) aus Kunststoff besteht, wobei in dem Zylindermantel (13) des Zylinders (9) Öffnungen (17) vorgesehen sind und der Zylinder (9) zumindest im Bereich einer Stirnseite Verankerungselemente (22, 30), beispielsweise ein im Abstand von der Stirnwand (10) angeordneter oder im Bereich der Stirnwand (10) gegenüber dem Zylindermantel vorspringend angeordneter Ring (32) zur Verankerung vorgesehen ist.

11. Vorrichtung nach Anspruch 10, dadurch gekennzeichnet, daß der Zylinder (9) an einer Stirnseite geöffnet und an der anderen Stirnseite durch die Stirnwand (10) verschlossen ist.

12. Vorrichtung nach einem der Ansprüche 1 bis 11, dadurch gekennzeichnet, daß die Stirnwand (10) des Mitnehmers (8) einen Durchmesser (16) aufweist, der größer ist als der äußere Durchmesser (12) des Zylinders (9), jedoch kleiner als der Durchmesser (7) des Tragkörpers (2).

13. Vorrichtung nach einem der Ansprüche 1 bis 12, dadurch gekennzeichnet, daß der Mitnehmer (8) aus einem elastomeren Kunststoff, insbesondere einem elastischen Silikonkautschuk und bzw. oder Weich-PVC und bzw. oder Pharmagummi und bzw. oder thermoplastischem Kautschuk besteht.

14. Vorrichtung nach einem der Ansprüche 1 bis 13, dadurch gekennzeichnet, daß der innere Durchmesser (16) des im Tragkörper (2) angeordneten Sackloches im wesentlichen dem inneren Durchmesser des als Zylinder (9) ausgebildeten Mitnehmers (8) entspricht und daß die Sacklochtiefe im wesentlichen der Gesamthöhe des den Mitnehmer (8) bildenden Zylinders (9) entspricht.

15. Vorrichtung nach einem der Ansprüche 1 bis 14, dadurch gekennzeichnet, daß die Höhe (5) des Tragkörpers (2) nur einen Teil des Durchmessers (7) desselben beträgt und größer ist als die Gesamthöhe (25) des als Mitnehmer (8) dienenden Zylinders (9).

16. Vorrichtung nach einem der Ansprüche 1 bis 15, dadurch gekennzeichnet, daß der Durchmesser (16) des Sackloches und bzw. oder des den Mitnehmer (8) bildenden Zylinders (9) kleiner ist als die Hälfte des Durchmessers (7) des Tragkörpers (2).

17. Vorrichtung nach einem der Ansprüche 1 bis 16, dadurch gekennzeichnet, daß der Mitnehmer durch ein Netz aus einem elastomeren Kunststoff oder dgl. gebildet ist und dieses Netz etwa in halber Höhe der Öffnung in den Tragkörper eingeschäumt ist.

18. Vorrichtung nach einem der Ansprüche 1 bis 17, dadurch gekennzeichnet, daß der Durchmesser der Öffnung (17, 29) und bzw. oder der innere Durchmesser des den Mitnehmer (8) bildenden Zylinders (9) in etwa dem durchschnittlichen Durchmesser eines menschlichen Fingers entspricht.

19. Vorrichtung nach einem der Ansprüche 1 bis 17, dadurch gekennzeichnet, daß die Maschenweite des den Mitnehmer (8) bildenden Netzes im wesentlichen dem durchschnittlichen Durchmesser eines menschlichen Fingers entspricht.

20. Vorrichtung nach einem der Ansprüche 1 bis 19, dadurch gekennzeichnet, daß die der konkaven Stirnfläche (62) gegenüberliegende Stirnfläche des aus porösem Kunststoff bestehenden Tragkörpers (61) mit einem als Folie (63), Film oder Scheibe ausgebildeten als Mitnehmer dienenden Teil aus elastomermem Material zumindest am Umfang mit dem Tragkörper (61) unlösbar verbunden, insbesondere verklebt ist.

21. Vorrichtung nach einem der Ansprüche 1 bis 20, dadurch gekennzeichnet, daß der mit dem Tragkörper (61) verbundene als Mitnehmer dienende Teil aus elastischem Silikonkautschuk und/oder Weich-PVC und/oder Pharmagummi besteht und mit dem aus Polyurethanweichschaum bestehenden Tragkörpers (61) mittels eines pharmakologisch unbedenklichen Klebers unlösbar verbunden ist.

22. Vorrichtung nach einem der Ansprüche 1 bis 21, dadurch gekennzeichnet, daß die Klebefläche zwischen dem Tragkörper (61) und dem als Mitnehmer dienenden Teil konkav ausgebildet ist.

23. Vorrichtung nach einem der Ansprüche 1 bis 22, dadurch gekennzeichnet, daß der poröse Tragkörper metallische Fäden aus Cu oder Ag enthält.

24. Vorrichtung nach einem der Ansprüche 1 bis 23, dadurch gekennzeichnet, daß Ränder (65) des die Stirnfläche des Tragkörpers abdeckenden Teiles um 180° einwärts gebogen und mit der Stirnfläche des Tragkörpers (61) verklebt sind.

25. Vorrichtung nach einem der Ansprüche 1 bis 24, dadurch gekennzeichnet, daß der Tragkörper (61) an seiner mit dem abdeckenden Teil verbundenen Stirnfläche eine mit der Zylinderachse des Tragkörpers (61) koaxiale Ausnehmung bzw. Vertiefung oder Öffnung (64) aufweist und daß der abdeckende Teil über die gesamte Stirnfläche des Tragkörpers mit diesem verklebt ist.

26. Vorrichtung nach einem der Ansprüche 1 bis 25, dadurch gekennzeichnet, daß die Ausnehmung bzw. Öffnung (64) wenigstens eine in Umfangsrichtung verlaufende Nut (65) aufweist, die von einwärts vorspringenden Kanten begrenzt und bzw. oder im Axialschnitt C-förmig ausgebildet ist.

27. Manipuliervorrichtung mit einer Schubstange zum intravaginalen Positionieren der Vorrichtung nach einem der Ansprüche 25 oder 26, dadurch gekennzeichnet, daß ein Ende der Schubstange (37) mit einem insbesondere pilzförmigen Ansatz (43) versehen ist, an dem sich in Richtung des anderen Endes der Schubstange (37) über einen Teilbereich seiner Länge ein verjüngter Bereich (44) mit geringerem Durchmesser anschließt und daß vorzugsweise die Schubstange (37) beidseits des verjüngten Bereiches (44) in etwa den gleichen Durchmesser aufweist und daß das Führungsrohr (36) in dem hinteren Endbereich einen Flansch (40) zur Aufnahme eines gegengleichen Anschlußstückes des Trichters (35) aufweist, wobei der innere Durchmesser des Flansches (40) um eine doppelte Wandstärke (41) des Anschlußstückes des Trichters (35) größer ist, als der innere Durchmesser (30) des Anschlußstückes des Trichters (35) und daß sich der innere Durchmesser (39) des Führungsrohres (36) in Richtung des vorderen Endbereiches verjüngt und insbesondere in Art eines Kegelabschnittes ausgebildet ist, wobei das Führungsrohr (36) von dem Flansch (40) bis in den gegenüberliegenden Endbereich einen konstanten äußeren Durchmesser aufweist.

28. Manipuliervorrichtung nach Anspruch 27, dadurch gekennzeichnet, daß der Durchmesser der Schubstange (37) in etwa dem inneren Durchmesser des Zylinders (9, 27) entspricht.

29. Manipuliervorrichtung nach einem der Ansprüche 27 oder 28, dadurch gekennzeichnet, daß der Trichter (35) in dem in das Führungsrohr (36) einsteckbaren Ansatzteil einen inneren Durchmesser (39) aufweist, der dem inneren Durchmesser (39) des Führungsrohres (36) entspricht, und sich in Richtung der dem einsteckbaren Ansatzteil gegenüberliegenden Stirnseite des Trichters über einen kegelstumpfförmigen Übergangsteil ein rohrförmiger Teil anschließt, der einen inneren Durchmesser (38) aufweist, der im wesentlichen dem äußeren Durchmesser (7) des Tragkörpers (2) entspricht.

30. Manipuliervorrichtung nach einem der Ansprüche 27 bis 29, dadurch gekennzeichnet, daß auf dem verjüngten Bereich (44) zugewendeten Seite des pilzförmigen Ansatzes (47) der Schubstange zumindest zwei Mitnehmer-Noppen (48) angeordnet sind.

31. Manipuliervorrichtung nach einem der Ansprüche 27 bis 30, dadurch gekennzeichnet, daß der stirnseitig offene rohrförmige Führungsteil (66) für die Aufnahme der zu positionierenden Vorrichtung bzw. des Schwammes in komprimierter Form in einem Endbereich einen geringeren Durchmesser (77) aufweist als sein Innendurchmesser (78) und daß ein Schieber (72) für den Ausstoß der zu positionierenden Vorrichtung (76) bzw. des Schwammes einen Verschlußteil (75) und durch das rohrförmige Führungsteil (66) durchführbare Klauen (70) aufweist, welche von einer dem Endbereich mit geringerem Durchmesser (77) gegenüberliegenden Seite her betätigbar sind und in einem entspannten Zustand einen geringeren Außendurchmesser (80) aufweisen als der Durchmesser (77) und mit den Nuten (65) der der Vorrichtung (76) bzw. des Schwammes in Eingriff bringbar ist.

32. Manipuliervorrichtung nach Anspruch 31, dadurch gekennzeichnet, daß eine Stirnfläche des Schiebers (72) ungefähr der lichten Querschnittsfläche des Führungsteiles (66) entspricht und die andere Stirnfläche einen auf kleineren Durchmesser abgesetzten Fortsatz (71) trägt, daß ein den Schieber (72) übergreifender zweiter rohrförmiger Teil (68) vorgesehen ist, dessen Außendurchmesser kleiner ist als der Innendurchmesser des rohrförmigen Führungsteiles (66), daß der zweite rohrförmige Teil (68) einen rohrförmigen Ansatz (69) trägt, an dessen freiem Ende nach außen verformbare Klauen (70) angeordnet sind, wobei der Innendurchmesser des rohrförmigen Ansatzes (69) größer ist als der Außendurchmesser des Fortsatzes (71) des Schiebers (72) und daß der rohrförmige Ansatz (6) sich im Bereich der Klauen (70) an seiner Innenseite auf einen Innendurchmesser verjüngt, welcher kleiner ist als der Außendurchmesser des Fortsatzes (71) des Schiebers (72).

## Claims

1. An intravaginally positionable and in particular contraceptive device (1) comprising an elastic carrying member (2) receiving a medication and formed by a substantially cylindrical block of plastics material which is provided with an opening (15) extending concentrically with respect to the cylinder axis (6), characterised in that the carrying member (2) is formed from a plastics material foam (3) and the opening (15) is produced in the form of a blind hole wherein an entraining element (8) is situated which is joined to the carrying member (2) by means of an expanding operation.

2. A device according to claim 1, characterised in that the entraining element (8) is co-expanded into the opening (15) having the form of a blind hole with a surface facing towards the carrying member (2).

3. A device according to claim 1 or 2, characterised in that the carrying member (2) is formed from an open-pored plastics material foam (3) which preferably also has open pores in the region of the surface.

4. A device according to claim 3, characterised in that the open-pored plastics material foam (3) has a soft foam of polyurethane.

5. A device according to claims 1 to 4, characterised in that the cells are closed at the side oppositely situated to the opening (15) having the form of a blind hole, in particular by appropriate temperature control during the foaming operation.

6. A device according to one of the claims 1 to 5, characterised in that the carrying member (2) is of one-piece construction.

7. A device according to one of the claims 1 to 6, characterised in that the end side (14) of the carrying member (2) lying opposite to the opening (15) is concavely formed and the transition between this concave end side (14) and a jacket surface (18) of the carrying member (2) is rounded off.

8. A device according to claim 7, characterised in that the transition between the end and jacket surfaces (14, 18) forms an encircling edge roll (19).

9. A device according to one of the claims 1 to 8, characterised in that the depth (24) of the curvature of the concave end face (14) is smaller than the height (5) of the carrying member (2) minus the overall height (25) of the cylinder (9) forming the entraining element.

10. A device according to one of the claims 1 to 9, characterised in that the entraining member (8) consists of a cylinder (9) of plastics material, openings (17) being provided in the cylinder jacket (13) of the cylinder (9) and the cylinder (9) has anchoring elements (22, 30) at least in the area of one end side, a ring (32) situated at a distance from the end side (10) or projectingly arranged with respect to the cylinder jacket in the area of the end side (10), for example being provided for anchoring purposes.

11. A device according to claim 10, characterised in that the cylinder (9) is open at one end side and closed by the end wall (10) at the other end side.

12. A device according to one of the claims 1 to 11, characterised in that the end wall (10) of the entraining element (8) has a diameter (16) which is greater than an outer diameter (12) of the cylinder (9) but smaller than the diameter (7) of the carrying member (2).

13. A device according to one of the claims 1 to 12, characterised in that the eintraining element (8) is formed from an elastomeric plastics material, in particular an elastic silicone rubber and/or soft PVC and/or «pharma» rubber and/or thermoplastic rubber.

14. A device according to one of the claims 1 to 13, characterised in that the inner diameter (16) of the blind hole situated in the carrying member (2) corresponds substantially to the inner diameter of the entraining element (8) constructed as a cylinder (9) and that the blind hole depth substantially corresponds to the overall height of the cylinder (9) forming the entraining element (8).

15. Device according to one of the claims 1 to 14, characterised in that the height (5) of the carrying member (2) amounts to a part only of the diameter (7) of the same and is greater than the overall height (25) of the cylinder (9) acting as an entraining element (8).

16. Device according to one of the claims 1 to 15, characterised in that the diameter (16) of the blind hole and/or of the cylinder (9) forming the entraining element (8) is smaller than one half of the diameter (7) of the carrying member (2).

17. Device according to one of the claims 1 to 16, characterised in that the entraining element is formed by a net or grid of an elastomeric plastics material or the like and that this net is integrally coexpanded into the carrying member at approximately half the height of the opening.

18. Device according to one of the claims 1 to 17, characterised in that a diameter of the opening (17, 29) and/or the internal diameter of the cylinder (9) forming the entraining element (8), corresponds approximately to the average diameter of a human finger.

19. Device according to one of the claims 1 to 17 characterised in that the mesh width of the net forming the entraining element corresponds substantially to the average diameter of a human finger.

20. Device according to one of the claims 1 to 19, characterised in that the end face oppositely situated to the concave end face (62) of the carrying member (61) formed from porous plastics material is indissolubly joined and in particular bonded, at least at the periphery, to the carrying member (6) via an element of elastomeric material formed as a foil (63), film or panel.

21. Device according to one of the claims 1 to 20 characterised in that the element joined to the carrying member (61) and acting as an entraining element consists of elastic silicone rubber and/or soft PVC and/or «pharma» rubber, and is indissolubly joined to the carrying member (61) consisting of soft polyurethane foam by means of a pharmacologically safe adhesive.

22. Device according to one of the claims 1 to 20, characterised in that the bonding area between the carrying member (61) and the element acting as an entraining element is concavely formed.

23. Device according to one of the claims 1 to 22, characterised in that the porous carrying member contains metal filaments of Cu or Ag.

24. Device according to one of the claims 1 to 23, characterised in that rims (65) of the element covering the end face of the carrying member are bent inwards through 180° and glued to the end face of the carrying member (61).

25. Device according to one of the claims 1 to 24, characterised in that the carrying member (61) has a recess or depression or opening (64) coaxial with the cylinder axis of the carrying member (61) at its end side joined to the covering element, and that the covering element is glued to the end side of the carrying member throughout its end side.

26. Device according to one of the claims 1 to 25, characterised in that the recess or opening (64) has at least one peripherally extending groove (65) which is delimited by inwardly projecting edges and/or is formed to C-shape in axial cross-section.

27. Handling device comprising a push rod for application of the method according to one of the claims 25 or 26, characterised in that one extremity of the push rod (37) is provided with an extension (43) of mushroom-shape in particular, which is followed in the direction of the other extremity of the push rod (37) and along a part section of its length by a reduced section (44) of lesser diameter and that the push rod (37) preferably has approximately the same diameter at either side of the reduced section (44), and that the push rod (37) preferably has approximately the same diameter at either side of the reduced section (44) and that the guiding tube (36) has a flange (40) in the rear terminal area for reception of a mating connector element for the funnel (35), the internal diameter of the flange (40) being greater by double the wall thickness (41) of the connector element of the funnel (35), than the internal diameter (30) of the connector element of the funnel (35), and that the internal diameter (39) of the guiding tube (36) diminishes in the direction of the front terminal section and in particular is constructed in the matter of a cone frustum, the guiding tube (36) having a constant external diameter from the flange (40) to the oppositely situated terminal portion.

28. Handling device according to claim 27, characterised in that the diameter of the push rod (37) corresponds approximately to the internal diameter of the cylinder (9, 27).

29. Handling device according to one of the claims 27 or 28, characterised in that the funnel (35) has an internal diameter (39) in the extension section insertible into the guiding tube (36), which corresponds to the internal diameter (39) of the guiding tube (36) and that a tubular element having an internal diameter (38) which corresponds substantially to the external diameter (7) of the carrying member (2) is connected in the direction if the end side of the funnel oppositely situated to the insertible extension section via a frustoconical transition portion.

30. Handling device according to one of the claims 27 to 29, characterised in that at least two entraining lugs (48) are situated on the side of the mushroomshaped extension (47) of the thrust or push rod facing towards the reduced section (44).

31. Handling device according to one of the claims 27 to 30, characterised in that the terminally open tubular guiding element (66) has a smaller diameter (77) in a terminal portion than its internal diameter (78) for reception in compressed form of the device or sponge which is to be positioned, and that a push rod (72) for ejection of the device (76) or sponge which is to be positioned has a closing element (75) and catches (70) apt to be passed through the tubular guiding element (66), which may be operated from a side situated opposite to the terminal section of lesser diameter (77) and in a relaxed condition have a smaller external diameter (80) than the diameter (77) and may be placed in engagement with the grooves (65) of the device (76) or sponge.

32. Handling device according to claim 31, characterised in that one end face of the push rod (72) corresponds approximately to the unobstructed cross-sectional area of the guiding element (66) and the other end face carries a projection (71) necked down to a smaller diameter, that a second tubular element (68) engaging over thrust element (72) is provided, whereof the external diameter is smaller than the internal diameter of the tubular guiding element (66), that the second tubular element (68) carries a tubular projection (69) on whose free extremity are situated outwardly deformable catches (70), the internal diameter of the tubular projection (69) being greater than the external diameter of the projection (71) of the thrust element (72) and that the tubular projection (69) is reduced on its inner side in the region of the catches (70) to an internal diameter which is smaller than the external diameter of the projection (71) of the thrust element (72).


**Revendications**

1. Dispositif intravaginal, notamment pour la contraception (1), avec un corps porteur élastique (2) se composant d'un bloc essentiellement cylindrique en matière synthétique, recevant un médicament (4), lequel corps est pourvu d'une ouverture (15) passant concentriquement à l'axe (6) du cylindre, caractérisé en ce que le corps porteur (2) est formé d'une mousse de matière synthétique (3) et l'ouverture (15) est en forme de trou borgne et dans celle-ci est disposé un entraîneur (8) qui est relié au corps porteur (2) par de la mousse.

2. Dispositif selon la revendication 1, caractérisé en ce que l'entraîneur (8) est pris dans la mousse par une surface supérieure tournée vers le corps porteur (2), dans l'ouverture (15) en forme de trou borgne.

3. Dispositif selon la revendication 1 ou 2, caractérisé en ce que le corps porteur (2) se compose d'une mousse en matière synthétique (3) à pores ouverts, qui présente avantageusement des cellules ouvertes également dans la zone de la surface supérieure.

4. Dispositif selon la revendication 3, caractérisé en ce que la mousse en matière synthétique (3) à pores ouverts est une mousse souple de polyuréthane.

5. Dispositif selon l'une des revendications 1 à 4, caractérisé en ce que du côté opposé à l'ouverture (15) en forme de trou borgne, les cellules sont fermées en particulier par réglage correspondant de la température pendant le processus de formation de mousse.

6. Dispositif selon l'une des revendications 1 à 5, caractérisé en ce que le corps porteur (2) est formé en une partie.

7. Dispositif selon l'une des revendications 1 à 6, caractérisé en ce que la surface frontale (14) du corps porteur (2) qui est opposée à l'ouverture (15) est concave et la transition entre cette surface frontale concave (14) et une surface d'enveloppe (18) du corps porteur (2) est arrondie.

8. Dispositif selon la revendication 7, caractérisé en ce que la transition entre les surfaces frontale et d'enveloppe (14, 18) forme un bourrelet continu (19).

9. Dispositif selon l'une des revendications 1 à 8, caractérisé en ce que la profondeur (24) du renflement de la surface frontale concave (14) est plus faible que la hauteur (15) du corps porteur (2), déduction faite de la hauteur totale (25) du cylindre (9) formant l'entraîneur (8).

10. Dispositif selon l'une des revendications 1 à 9, caractérisé en ce que l'entraîneur (8) se compose d'un cylindre (9) en matière synthétique, et dans l'enveloppe (13) du cylindre (9) sont prévues des ouvertures (17) et le cylindre (9) est pourvu, au moins dans la zone d'un côté frontal, d'éléments d'ancrage (22, 30), par exemple une bague (32) est prévue pour l'ancrage, disposée à une certaine distance de la paroi frontale (10) ou disposée dans la zone de la paroi frontale (10), dépassant de l'enveloppe du cylindre.

11. Dispositif selon la revendication 10, caractérisé en ce que le cylindre (9) est ouvert à un côté frontal et à l'autre côté frontal est fermé par la paroi frontale (10).

12. Dispositif selon l'une des revendications 1 à 11, caractérisé en ce que la paroi frontale (10) de l'entraîneur (8) présente un diamètre (16) qui est plus grand que le diamètre externe (12) du cylindre (9), mais qui est cependant plus petit que le diamètre (7) du corps porteur (2).

13. Dispositif selon l'une des revendications 1 à 12, caractérisé en ce que l'entraîneur (8) se compose d'une matière synthétique élastomère, en particulier d'un caoutchouc élastique de silicone et/ou de PVC souple et/ou de cautchouc pharmaceutique et/ou de cautchouc thermoplastique.

14. Dispositif selon l'une des revendications 1 à 13, caractérisé en ce que le diamètre interne (16) du trou borgne placé dans le corps porteur (2) correspond essentiellement au diamètre interne de l'entraîneur (8) configuré en cylindre (9) et en ce que la profondeur du trou borgne correspond essentiellement à la hauteur totale du cylindre (9) formant l'entraîneur (8).

15. Dispositif selon l'une des revendications 1 à 14, caractérisé en ce que la hauteur (5) du corps porteur (2) ne représente qu'une partie de son diamètre (7) et est plus grande que la hauteur totale (25) du cylindre (9) servant d'entraîneur (8).

16. Dispositif selon l'une des revendications 1 à 15, caractérisé en ce que le diamètre (16) du trou borgne et/ou du cylindre (9) formant l'entraîneur (8) est plus petit que la moitié du diamètre (7) du corps porteur (2).

17. Dispositif selon l'une des revendications 1 à 16, caractérisé en ce que l'entraîneur est formé d'un filet en une matière synthétique élastomère ou analogue et ce filet est enfoncé dans la mousse à peu près à mi-hauteur de l'ouverture dans le corps porteur.

18. Dispositif selon l'une des revendications 1 à 17, caractérisé en ce que le diamètre de l'ouverture (17, 29) et/ou le diamètre interne du cylindre (9) formant l'entraîneur (8) correspond à peu près au diamètre normal d'un doigt d'être humain.

19. Dispositif selon l'une des revendications 1 à 17, caractérisé en ce que la largeur des mailles du filet formant l'entraîneur (8) correspond essentiellement au diamètre moyen d'un doigt d'être humain.

20. Dispositif selon l'une des revendications 1 à 19, caractérisé en ce que la surface concave opposée à la surface concave frontale (62) du corps porteur (61) se composant d'une matière synthétique poreuse est reliée de manière non détachable, en particulier est collée au moins périphériquement au corps porteur (61) au moyen d'une partie en matériau élastomère servant d'entraîneur et configurée en une feuille (63), un film ou un disque.

21. Dispositif selon l'une des revendications 1 à 20, caractérisé en ce que la partie servant d'entraîneur reliée au corps porteur (61) se compose d'un caoutchouc élastique de silicone et/ou de PVC souple et/ou de caoutchouc de pharmacie et est reliée au corps porteur (61) se composant d'une mousse souple de polyuréthane au moyen d'une colle pharmacologiquement inoffensive, de manière non détachable.

22. Dispositif selon l'une des revendications 1 à 21, caractérisé en ce que la surface de colle entre le corps porteur (61) et la partie servant d'entraîneur est de forme concave.

23. Dispositif selon l'une des revendications 1 à 22, caractérisé en ce que le corps porteur poreux contient des fils métalliques en Cu ou Ag.

24. Dispositif selon l'une des revendications 1 à 13, caractérisé en ce que des bords (65) de la partie recouvrant la surface frontale du corps porteur sont courbés vers l'intérieur sur 180° et sont collés à la surface frontale du corps porteur (61).

25. Dispositif selon l'une des revendications 1 à 24, caractérisé en ce que le corps porteur (61), à sa surface frontale reliée à la partie recouvrante présente un évidement ou respectivement renfoncement ou ouverture (64) coaxial avec l'axe du cylindre du corps porteur (61) et en ce que la partie recouvrante est collée à celui-ci par toute la surface frontale du corps porteur.

26. Dispositif selon l'une des revendications 1 à 25, caractérisé en ce que l'évidement ou respectivement ouverture (64) présente au moins une rainure en direction périphérique (65) qui est délimitée par des arêtes dépassant vers l'intérieur et/ou est en forme de C en coupe axiale.

27. Dispositif de manipulation avec une tige de poussée pour le positionnement intravaginal du dispositif selon l'une des revendications 25 ou 26, caractérisé en ce qu'une extrémité de la tige de poussée (37) est pourvue d'une saillie (43) en particulier en forme de champignon, à laquelle se raccorde, dans la direction de l'autre extrémité de la tige de poussée (37), sur une partie de sa longueur, une zone rétrécie (44) d'un diamètre moindre et en ce qu'avantageusement la tige de poussée (37) présente, des deux côtés de la zone rétrécie (44), à peu près le même diamètre et en ce que le tube de guidage (36) présente, à sa partie extrême arrière, une bride (40) pour la réception d'une pièce diamétralement opposée de raccordement de l'entonnoir (35), le diamètre interne de la bride (40) étant plus grand d'une double épaisseur de paroi (41) de la pièce de raccordement de l'entonnoir (35) que le diamètre interne (30) de la pièce de raccordement de l'entonnoir (35) et en ce que le diamètre interne (39) du tube de guidage (36) se rétrécit dans la direction de la partie extrême avant et en particulier a la forme d'un tronc de cône, le tube de guidage (36) ayant, de la bride (40) jusqu'à la partie extrême opposée, un diamètre externe constant.

28. Dispositif de manipulation selon la revendication 27, caractérisé en ce que le diamètre de la tige de poussée (37) correspond à peu près au diamètre interne du cylindre (9, 27).

29. Dispositif de manipulation selon l'une des revendications 27 ou 28, caractérisé en ce que l'entonnoir (35) présente, dans sa partie pouvant s'enfoncer dans le tube de guidage (36), un diamètre interne (39) qui correspond au diamètre interne (39) du tube de guidage (36) et se raccorde, dans la direction du côté frontal de l'entonnoir, opposé à la partie d'insertion, par une partie de transition en forme de tronc de cône, à une partie de forme tubulaire, qui présente un diamètre interne (38) qui correspond essentiellement au diamètre externe (7) du corps porteur (2).

30. Dispositif de manipulation selon l'une des revendications 27 à 29, caractérisé en ce que sur le côté de la saillie en forme de champignon (47) de la tige de poussée qui est tourné vers la zone rétrécie (44), sont disposées au moins deux noppes d'entraîneur (48).

31. Dispositif de manipulation selon l'une des revendications 27 à 30, caractérisé en ce que la partie de guidage (66) en forme de tube ouvert du côté frontal présente, pour la réception du dispositif à positionner, ou respectivement de l'éponge sous une forme comprimée, dans une partie extrême, un diamètre (77) plus petit que son diamètre interne (78) et en ce qu'un poussoir (72) pour l'expulsion de dispositif (76) à positionner ou respectivement de l'éponge présente une partie d'obturation (75) et des pattes (70) pouvant être guidées à travers la partie tubulaire de guidage (66), lesquelles peuvent être actionnées à partir du côté se trouvant face à la partie extrême de moindre diamètre (77) et présentent, à un état détendu, un diamètre externe (80) plus petit que le diamètre (77) et peuvent être mises en engagement avec les rainures (65) du dispositif (76) ou respectivement de l'éponge.

32. Dispositif de manipulation selon la revendication 31, caractérisé en ce qu'une surface frontale du poussoir (72) correspond à peu près à la section transversale interne de la partie de guidage (66) et l'autre surface frontale porte une saillie (71) d'un plus petit diamètre, en ce qu'une seconde partie tubulaire (68) est prévue qui passe sur le poussoir (72), dont le diamètre externe est plus petit que le diamètre interne de la partie de guidage tubulaire (66), en ce que la seconde partie tubulaire (68) porte une saillie tubulaire (69) à l'extrémité libre de laquelle sont disposées des pattes déformables vers l'extérieur (70), le diamètre interne de la saillie tubulaire (68) étant plus gros que le diamètre externe de la saillie (71) du poussoir (72) et en ce que la saillie tubulaire (6) se rétrécit dans la zone des pattes (70), à son côté interne, à un diamètre interne qui est plus petit que le diamètre externe de la saillie (71) du poussoir (72).

*Fig. 1*

*Fig. 2*

*Fig. 3*

_Fig. 4_

_Fig. 5_

Fig. 6

Fig. 7

Fig. 8

Fig. 9

Fig. 13

Fig. 15

Fig. 12

Fig. 14

Fig. 17

Fig. 11

Fig. 10

Fig. 16